# EUROPEAN PATENT SPECIFICATION

(11) **EP 1 734 896 B1**
(45) Date of publication and mention of the grant of the patent: **26.03.2008**
(21) Application number: 05731288.6
(22) Date of filing: 18.04.2005
(51) Int. Cl.: A61F 2/01

(54) **A SELF CENTERING VENA CAVA FILTER**
SELBSTZENTRIERENDER VENA CAVA FILTER
FILTRE DE VEINE CAVE A AUTOCENTRAGE

(30) Priority: 16.04.2004 US 562809 P; 16.04.2004 US 563243 P; 16.04.2004 US 563171 P; 16.04.2004 US 562943 P; 16.04.2004 US 562813 P; 16.04.2004 US 562909 P; 16.04.2004 US 563176 P; 16.04.2004 US 563192 P
(43) Date of publication of application: 27.12.2006
(73) Proprietor: WILLIAM COOK EUROPE ApS, 4632 Bjaeverskov (DK); Cook Incorporated, Bloomington, Indiana 47402 (US)
(72) Inventor: MØLGAARD-NIELSEN, Arne, DK-2100 Copenhagen ø (DK); HENDRIKSEN, Per, DK-4850 Stubbekøbing (DK); HEMMINGSEN, Allan, DK-2650 Hvidovre (DK); OSBORNE, Thomas A., Bloomington, IN 47401 (US)
(74) Representative: Garratt, Peter Douglas
(86) International application number: PCT/DK2005/000265
(87) International publication number: WO 2005/099620

(56) References cited:
- WO-A-20/04049973
- FR-A- 2 672 487
- US-A- 4 781 177
- US-A- 5 836 968
- US-A1- 2004 186 510
- US-B1- 6 391 045
- US-B1- 6 468 290

## Description

### BACKGROUND OF THE INVENTION

The present invention relates to medical devices. More particularly, the invention relates to a removable vena cava clot filter that can be percutaneously placed in and removed from the vena cava of a patient.

Filtering devices that are percutaneously placed in the vena cava have been available for over thirty years. A need for filtering devices arises in trauma patients, orthopedic surgery patients, neurosurgery patients, or in patients having medical conditions requiring bed rest or non-movement. During such medical conditions, the need for filtering devices arises due to the likelihood of thrombosis in the peripheral vasculature of patients wherein thrombi break away from the vessel wall, risking downstream embolism or embolization. For example, depending on the size, such thrombi pose a serious risk of pulmonary embolism wherein blood clots migrate from the peripheral vasculature through the heart and into the lungs.

A filtering device can be deployed in the vena cava of a patient when, for example, anticoagulant therapy is contraindicated or has failed. Typically, filtering devices are permanent implants, each of which remains implanted in the patient for life, even though the condition or medical problem that required the device has passed. In more recent years, filters have been used or considered in preoperative patients and in patients predisposed to thrombosis which places the patient at risk for pulmonary embolism.

The benefits of a vena cava filter have been well established, but improvements may be made. For example, filters generally have not been considered removable from a patient due to the likelihood of endotheliosis of the filter or fibrous reaction matter adherent to the endothelium during treatment. After deployment of a filter in a patient, proliferating intimal cells begin to accumulate around the filter struts which contact the wall of the vessel. After a length of time, such ingrowth prevents removal of the filter without risk of trauma, requiring the filter to remain in the patient. As a result, there has been a need for an effective filter that can be removed after the underlying medical condition has passed.

Moreover, conventional filters commonly become off-centered or tilted with respect to the hub of the filter and the longitudinal axis of the vessel in which it has been inserted. As a result, the filter including the hub and the retrieval hook engage the vessel wall along their lengths and potentially become endothelialized therein. This condition is illustrated in prior art Figure la in which a prior art filter 113 has been delivered by a delivery sheath 125 through the vessel 150 of a patient. In the event of this occurrence, there is a greater likelihood of endotheliosis of the filter to the blood vessel along a substantial length of the filter wire. As a result, the filter becomes a permanent implant in a shorter time period than otherwise.

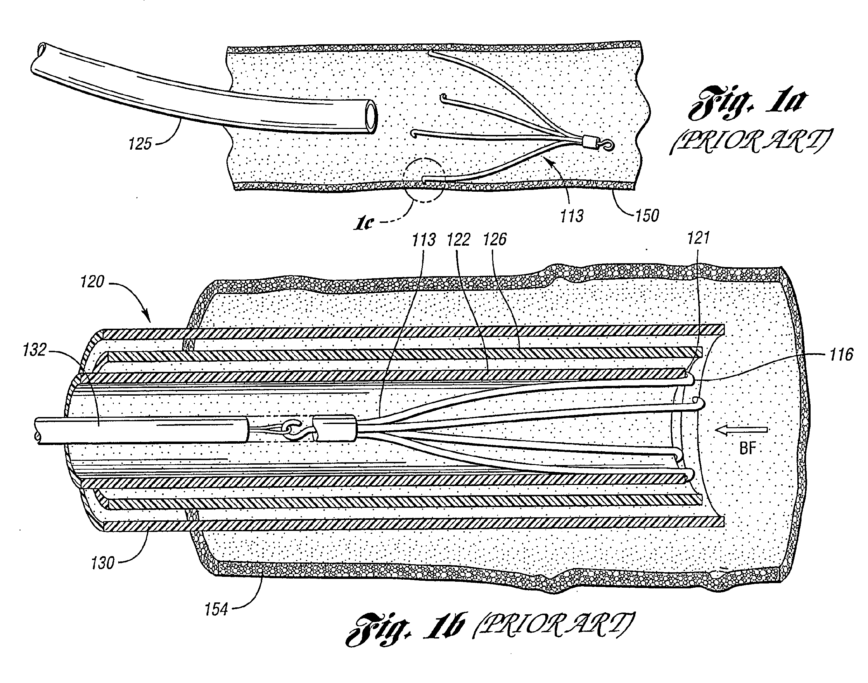

Furthermore, further improvements may be made related to the delivery or retrieval of vena cava filters. For delivery of vena cava filters, an introducer system having an introducer tube may be percutaneously inserted in the vena cava of a patient through the femoral vein or the jugular vein. A part of an introducer assembly 120 is illustrated in prior art Figure 1b in which the prior art filter 113 is percutaneously delivered through the jugular vein 154 of a patient. As shown, the filter 113 in its collapsed configuration is placed at the distal end 121 of an inner sheath 122 with anchoring hooks 116 of the filter 113 extending past the distal end 121. An outer sheath 126 is then disposed over the inner sheath 122 to avoid undesirably scratching or scraping of the anchoring hooks 116 against the introducer tube 130. The inner and outer sheaths 122, 126 along with a pusher member 132 are then moved together through the introducer tube 130 to deliver the filter 113 to the vena cava of the patient.

It has been a challenge to design a vena cava filter with features that lessen the concerns of undesirably scratching or scraping of the anchoring hooks against outer walls of an introducer tube or a blood vessel while maintaining the effectiveness of the filter.

### BRIEF SUMMARY OF THE INVENTION

One embodiment of the present invention according to claim 1 generally provides a removable vena cava filter configured for simplified delivery to and retrieval from the vena cava of a patient. The filter is shaped for easy delivery and retrieval. The filter includes primary and secondary struts, each having a first end and an arcuate segment extending therefrom arcuately along a longitudinal axis and linearly relative to a radial axis.

The present invention provides a removable vena cava filter having a collapsed state and an expanded state for capturing thrombi in a blood vessel. In one embodiment, the filter comprises a plurality of primary struts. Each primary strut in the expanded state extends from a primary strut first end to an anchoring hook. Each primary strut extends arcuately along a longitudinal axis and linearly radially. The plurality of primary strut first ends are attached together along the longitudinal axis. The filter further comprises a plurality of secondary struts. Each secondary strut in the expanded state extends from a secondary strut first end to a free end. Each secondary strut extends arcuately along the longitudinal axis and linearly radially. The plurality of secondary struts are attached together along the longitudinal axis. The plurality of secondary struts centralize the filter in the expanded state in the blood vessel.

In another embodiment, the removable vena cava filter includes a hub axially housing the primary strut first ends and secondary strut first ends. The filter further includes a retrieval hook extending from the hub opposite the plurality of primary struts for removal of the filter from the blood vessel.

In yet another embodiment, pairs of secondary struts are positioned between pairs of primary struts. Each pair of secondary struts is twisted together near the connected ends of the secondary struts to form a twisted section. The twisted sections of the secondary struts effectively stiffen the struts to enhance their centering capabilities to prevent the filter from tilting when the filter is deployed in the blood vessel. Hence, engagement between the struts and the blood vessel is minimized which reduces the potential for the struts to become endothelialized within the blood vessel. A further feature of the twisted sections is that they prevent or at least minimize the secondary struts from entangling with the primary struts.

Further aspects, features, and advantages of the invention will become apparent from consideration of the following description and the appended claims when taken in connection with the accompanying drawings.

### BRIEF DESCRIPTION OF THE DRAWINGS

Figure 1a is a side view of a prior art filter deployed through the vasculature of a patient;
Figure 1b is a side view of an introducer assembly including the prior art filter to be delivered to the vena cava of a patient;
Figure 2 is an illustration of the anatomy of the renal veins, the iliac veins, and the vena cava in which one embodiment of a vena cava filter of the present invention is deployed;
Figure 3a is a side perspective view of one embodiment of the vena cava filter in an expanded state;
Figure 3b is a side view of a primary strut of the filter in Figure 3a in accordance with one embodiment of the present invention;
Figure 3c is a side view of the vena cava filter of Figure 3a in a collapsed state and disposed in an introducer tube;
Figure 4 is an enlarged view of a portion of a second arcuate portion of a primary strut of the vena cava filter;
Figure 5 is a cross-sectional view of a hub of the filter in Figure 3 taken along line 5-5;
Figure 6a is a cross-sectional view of the vena cava depicting the filter partially deployed leading with the removal hook;
Figure 6b is a cross-sectional view of the vena cava depicting the filter partially deployed leading with the anchoring hooks;
Figure 7 is a cross-sectional view of the vena cava in which the filter of Figure 3a has been deployed;
Figure 8a is a cross-sectional view of the vena cava of Figure 7a taken along line 8-8;
Figure 8b is a cross-sectional view of the vena cava of Figure 7a taken along line 8-8 depicting another embodiment of the filter;
Figure 9a is a cross-sectional view of a blood vessel in which a retrieval sheath engages primary struts of the filter in Figure 3 for removal;
Figure 9b is a cross-sectional view of a blood vessel in which the retrieval sheath includes the filter in the collapsed state for removal;
Figure 10 is a cross-sectional view of a blood vessel showing a vena cava filter of deployed within the blood vessel in accordance with another embodiment of the invention; and
Figure 11 is a view of the blood vessel and filter of Figure 10 taken along the line 11-11.

### DETAILED DESCRIPTION OF THE INVENTION

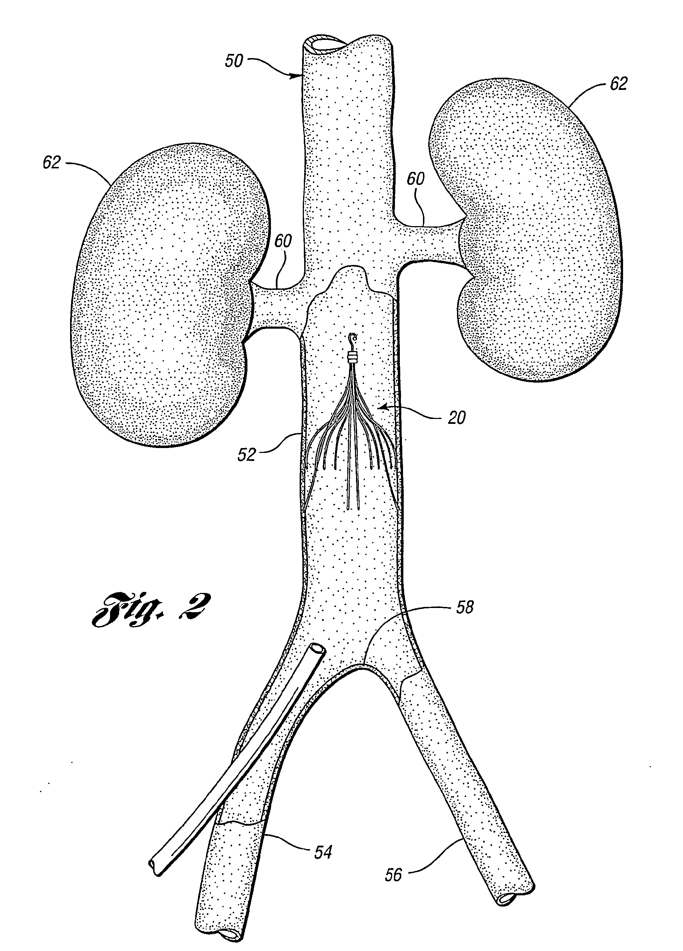

In accordance with one embodiment of the present invention, Figure 2 illustrates a vena cava filter 10 implanted in the vena cava 50 for the purpose of lysing or capturing thrombi carried by the blood flowing through the iliac veins 54, 56 toward the heart and into the pulmonary arteries. As shown, the iliac veins merge at juncture 58 into the vena cava 50. The renal veins 60 from the kidneys 62 join the vena cava 50 downstream of juncture 58. The portion of the vena cava 50, between the juncture 58 and the renal veins 60, defines the inferior vena cava 52 in which the vena cava filter 10 has been percutaneously deployed through one of the femoral veins. Preferably, the vena cava filter 10 has a length smaller than the length of the inferior vena cava 52. If the lower part of the filter extends into the iliac veins, filtering effectiveness will be compromised and if the filter wires cross over the origin of the renal veins the filter wires might interfere with the flow of blood from the kidneys.

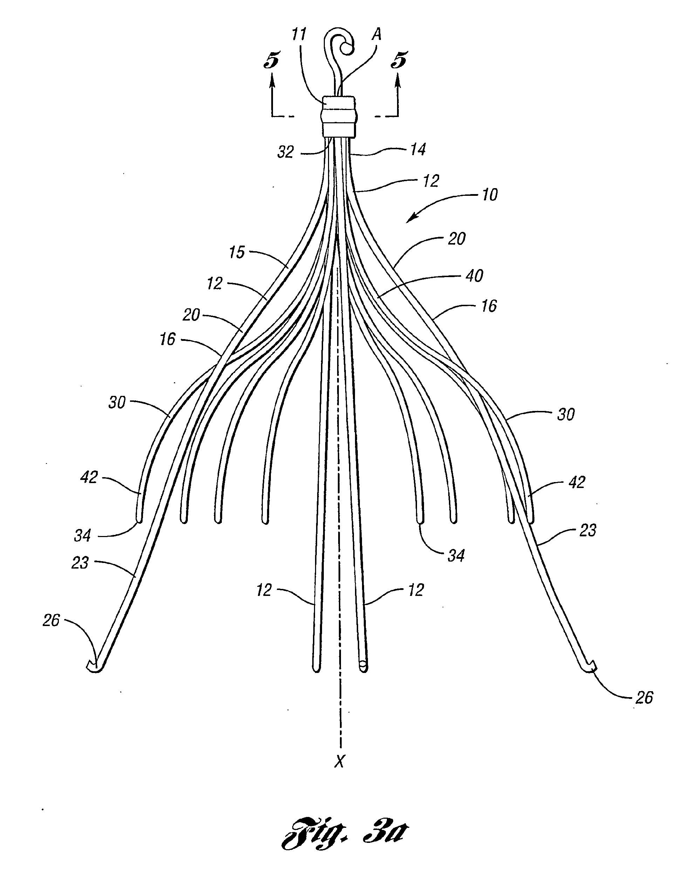

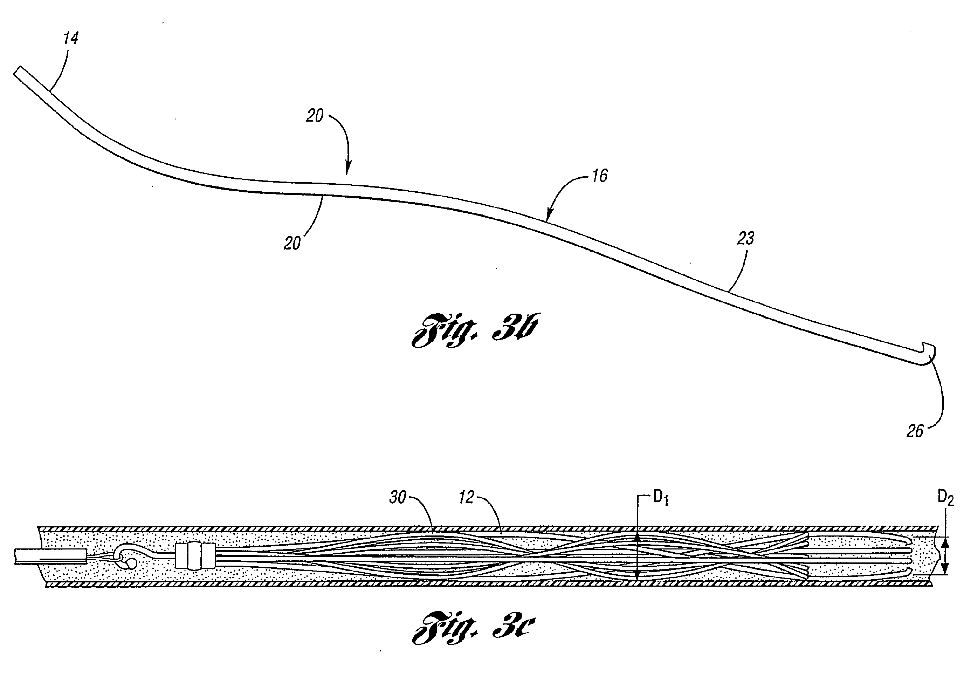

This embodiment of the present invention will be further discussed with reference to Figures 3-9 in which filter 10 is shown. Figure 3a illustrates filter 10 in an expanded state and comprising four primary struts 12 each having first ends that emanate from a hub 11. Hub 11 attaches by crimping first ends 14 of primary struts 12 together at a center point A in a compact bundle along a central or longitudinal axis X of the filter. The hub 11 has a minimal diameter for the size of wire used to form the struts.

Preferably, the primary struts 12 are formed of a superelastic material, stainless steel wire, Nitinol, cobalt-chromium-nickel-molybderium-iron alloy, or cobalt chrome-alloy or any other suitable superelastic material that will result in a self-opening or self-expanding filter. In this embodiment, the primary struts 12 are preferably formed from wire having a round cross-section with a diameter of at least about 0,381 mm (0.015 inches). Of course, it is not necessary that the primary struts have a round or near round cross-section. For example, the primary struts 12 could take on any shape with rounded edges to maintain non-turbulent blood flow therethrough.

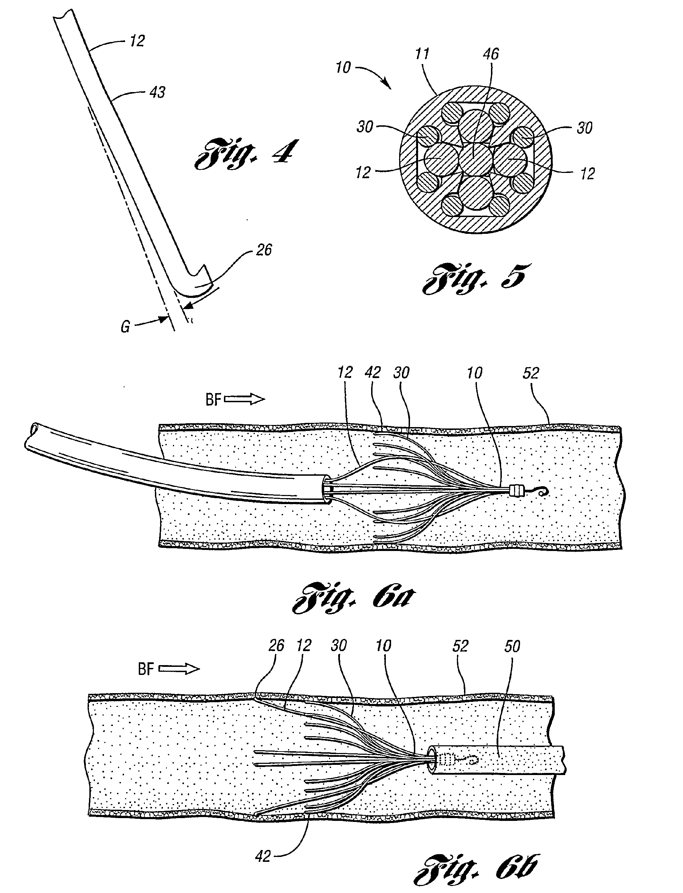

As shown in Figures 3a and 3b, each primary strut 12 includes an arcuate segment 16 having a soft S-shape. Each arcuate segment 16 is formed with a first curved portion 20 that is configured to softly bend away from the longitudinal or central axis X of the filter 10 and a second curved portion 23 that is configured to softly bend toward the longitudinal axis of the filter 10. In an embodiment, the primary struts 12 comprises a straight segment extending from a distal end 14 for about 5 mm for anchoring in the hub 11. The length of the first curved portion 20 could be about 8-12 mm and the radius of curvature about 60-80 mm. The length of the second curved portion 23 could be 8-15 mm and the radius of curvature about 40-60 mm. As explained in more detail below with reference to Fig. 4, a distal bend 43 is formed on the strut 12 extending outwardly radially from the longitudinal axis X. This distal bend 43 may have a length of 1-7 mm, preferably 2-4 mm, whereas a straight distal segment has a length of 15-25 mm. Due to the soft bends of each arcuate segment 16, a prominence or a point of inflection on the primary strut 12 is substantially avoided to aid in non-traumatically engaging the vessel wall.

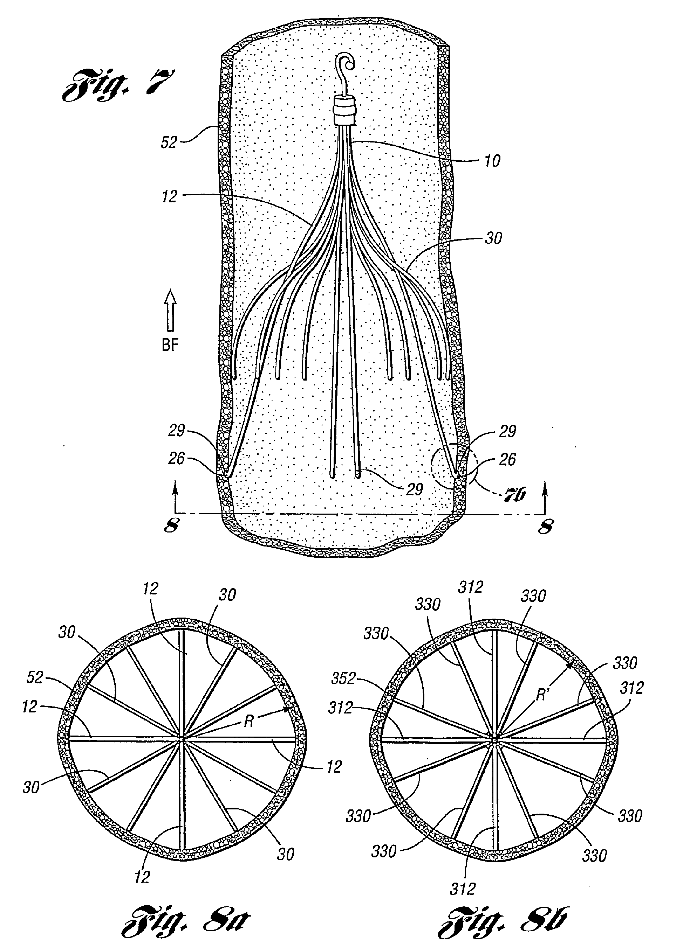

As shown in Figure 3b, the primary struts 12 terminate at anchoring hooks 26 that will anchor in the vessel wall when the filter 10 is deployed at a delivery location in the blood vessel. The primary struts 12 are configured to move between an expanded state for engaging the anchoring hooks 26 with the blood vessel and a collapsed state for filter retrieval or delivery. In the expanded state, each arcuate segment 16 extends arcuately along a longitudinal axis X (as shown in Figure 3a) and linearly relative to a radial axis R (as shown in Figure 8a) from the first end 14 to the anchoring hook 26. As shown in Figure 8a, the primary struts 12 radially extend from the first ends 14, defining the radial axis R. In this embodiment, the primary struts 12 extend linearly relative to the radial axis R and avoid entanglement with other struts.

As discussed in greater detail below, the soft bends of each arcuate segment 16 allow each primary strut 12 to cross another primary strut 12 along the longitudinal axis X in the collapsed state such that each anchoring hook 26 faces the longitudinal axis X for filter retrieval or delivery.

When the filter 10 is deployed in a blood vessel, the anchoring hooks 26 engage the walls of the blood vessel to define a first axial portion to secure the filter in the blood vessel. The anchoring hooks 26 prevent the filter 10 from migrating from the delivery location in the blood vessel where it has been deposited. The primary struts 12 are shaped and dimensioned such that, when the filter 10 is freely expanded, the filter 10 has a diameter of between about 25 mm and 45 mm and a length of between about 3 cm and 7 cm. For example, the filter 10 may have a diameter of about 35 mm and a length of about 5 cm. The primary struts 12 have sufficient spring strength that when the filter is deployed the anchoring hooks 26 will anchor into the vessel wall.

In this embodiment, the filter 10 includes a plurality of secondary struts 30 having connected ends 32 that also emanate from hub 11 as shown in Figure 3a. Hub 11 attaches by crimping the connected ends 32 at the center point A of the secondary struts 30 together with the primary struts 12. In this embodiment, each primary strut 12 has two secondary struts 30 in side-by-side relationship with the primary strut 12. The secondary struts 30 extend from the connected ends 32 to free ends 34 to centralize the filter 10 in the expanded state in the blood vessel. As shown, each secondary strut 30 extends arcuately along the longitudinal axis and linearly relative to the radial axis from the connected end 32 to the free end 34 for engaging the anchoring hooks 26 with the blood vessel. As with the primary struts 12, the secondary struts 30 extend linearly relative to the radial axis and avoid entanglement with other struts.

The secondary struts 30 may be made from the same type of material as the primary struts 12. However, the secondary struts 30 may have a smaller diameter, e.g., at least about 0,305 mm (0.012 inches), than the primary struts 12. In this embodiment, each of the secondary struts 30 is formed of a first arc 40 and a second arc 42. The first arc 40 extends from the connected end 32 away from the longitudinal axis X. The second arc 42 extends from the first arc 40 towards the longitudinal axis X. As shown, two secondary struts 30 are located on each side of one primary strut 12 to form a part of a netting configuration of the filter 10. The hub 11 is preferably made of the same material as the primary struts and secondary struts to minimize the possibility of galvanic corrosion or molecular changes in the material due to welding.

When freely expanded, free ends 34 of the secondary struts 30 will expand radially outwardly to a diameter of about 25 mm to 45 mm. For example, the secondary struts 30 may expand radially outwardly to a diameter of between about 35 mm and 45 mm. The second arcs 42 of the free ends 34 engage the wall of a blood vessel to define a second axial portion where the vessel wall is engaged. The secondary struts 30 function to stabilize the position of the filter 10 about the center of the blood vessel in which it is deployed.

As a result, the filter 10 has two layers or portions of struts longitudinally engaging the vessel wall of the blood vessel. The length of the filter 10 is preferably defined by the length of a primary strut 12. Furthermore, the diameter of the hub 11 is defined by the size of a bundle containing the primary struts 12 and secondary struts 30. In this embodiment, the eight secondary struts 30 minimally add to the diameter of the hub 11 or the overall length of the filter 10, due to the reduced diameter of each secondary strut 30. This is accomplished while maintaining the filter 10 in a centered attitude relative to the vessel wall and formed as a part of the netting configuration of the filter 10. As shown, removal hook 46 extends from hub 11 opposite primary and secondary struts 12 and 30.

In this embodiment, each arcuate segment 16 has a thickness of at least about 0,38 mm (0.015 inch) and a tensile strength of between about 2 GPa (285,000 pounds per square inch (psi)) and 2,3 GPa (330,000 psi). Each anchoring hook 26 is integral with the arcuate segment 16 and has the thickness and the tensile strength of the arcuate segment. Each secondary strut 30 has a thickness of at least about 0,305 mm (0.012 inch) and a tensile strength of between about 2 GPa (285,000 psi) and 2,3 GPa (330, 000 psi).

Figure 3c illustrates the filter 10 according to the invention in a collapsed state disposed in a delivery/retrieval tube 94 for delivery or retrieval. As shown, the filter 10 is shaped for each primary strut 12 to cross another primary strut 12 along the longitudinal axis X. As a result, in the collapsed state, the anchoring hooks 26 are configured to invert or inwardly face the longitudinal axis X for retrieval and delivery of the filter 10. This inverted or inwardly facing configuration of the anchoring hooks 26 allows for simplified delivery and retrieval of filter 10. For example, a concern that the anchoring hooks 26 may scrape, scratch, or tear the inner wall of a delivery/retrieval tube is eliminated, since the filter 10 of the present invention is shaped to have the anchoring hooks 26 face each other in the collapsed state. A pair of opposed primary struts may be off-set by bending the strut by 1-2° at right angles to the plane of the strut, i.e. at right angles to the plane of the paper in Fig. 3b, to allow the pair of struts to cross each other. In an embodiment, the off-set is provided by bending the strut at or near the region between the first and secondary curved sections 20, 23. By the off-set, two opposed primary struts 12 may extend substantially in parallel, when seen in the plane of each arcuately extending strut. In fact, a set of inner and outer delivery/retrieval sheaths (see prior art Figure 1b) may be eliminated during the delivery or retrieval of the filter 10 through the jugular or femoral vein. Rather, merely one delivery/retrieval tube with a loop snare mechanism may be used to deliver or retrieve the filter 10 of the present invention.

Moreover, in the collapsed state, each primary strut 12 is configured to cross another primary strut 12 along the longitudinal axis X such that the arcuate segments 16, first curved portions 20 or second curved portions 23, occupy a first diameter D1. In this embodiment, the first diameter is greater than a second diameter D2 occupied by the anchoring hooks 26 for filter retrieval or delivery. It has been found that the first diameter of the arcuate segments 16 serves to clear a path of retrieval, reducing radial force from the sheath or blood vessel on the anchoring hooks 26 during removal of the filter 10 from a patient. Reducing the radial force on the anchoring hooks 26 assists in preventing the anchoring hooks 26 from scraping, scratching, or tearing the inner wall of a sheath during removal of the filter 10 from a patient.

In this embodiment of the present invention, it is to be noted that the filter 10 may be delivered or retrieved by any suitable introducer (delivery or retrieval) tube. However, it is preferred that the introducer tube has an inside diameter of between about 4.5 French and 16 French, and more preferably between about 6.5 French and 14 French.

Figure 4 illustrates primary strut 12 including distal bend 43 formed thereon and extending outwardly radially from the longitudinal axis X. As shown in Figure 4, the distal bend 43 may extend outwardly at an angle between about 0.5 degree to 2 degrees, preferably 1.0 degree. The distal bend 43 may be situated at a distance from the anchoring hook 26, which is arranged at the end of a substantially straight strut segment. The distal bend 43 allows the filter 10 to filter thrombi effectively at a smaller inside diameter of a blood vessel than otherwise would be possible while maintaining the ability to collapse for delivery or retrieval. Further the distal bend 43 provides for a more firm engagement of the anchoring hook 26 at the vessel wall. At the engagement of the anchoring hook 26 with the vessel wall, the primary struts 12 will urge the vessel wall outwards, whereas the vessel wall will urge the primary struts 12 inwards toward the longitudinal axis X of the filter 10. In a preferred embodiment, the anchoring hooks 26 are angled by 50-80° in respect to the last segment of the primary strut 12, preferably 50-60°.

Figure 5 illustrates a cross-sectional view of the filter 10 of Figure 3a at hub 11. As shown, the hub 11 houses a bundle of first ends 14 of the four primary struts 14 and connected ends 32 of secondary struts 30. Figure 5 further depicts the configurations of the primary and secondary struts 12 and 30. In this embodiment, the primary struts 12 are spaced between two secondary struts 30. Of course, the primary struts 12 may be spaced between any other suitably desired number of secondary struts 30 without falling beyond the scope of the present invention.

In this embodiment, Figures 6a and 6b both illustrate the filter 10 partially deployed in inferior vena cava 52. Figure 6a shows the filter 10 being delivered by a delivery tube 48 through the vasculature of a patient and Figure 6b shows the filter 10 being delivered by a delivery tube 50 through the jugular vein of a patient. For deployment of the filter 10, a delivery tube is percutaneously inserted through the patient's vessel such that the distal end of the delivery tube is at the location of deployment. In this embodiment, a wire guide is preferably used to guide the delivery tube to the location of deployment. In Figure 6a, the filter 10 is inserted through the proximal end of the delivery tube 48 with the removal hook 46 leading and anchoring hooks 26 of the primary struts 12 held by a filter retainer member for delivery via the femoral vein of a patient.

In Figure 6b, the filter 10 is inserted through the proximal end of the delivery tube 50 with the anchoring hooks 26 of the primary struts 12 leading and the removal hook 46 trailing for delivery via the jugular vein of a patient. In this embodiment, a pusher wire having a pusher member at its distal end may be fed through the proximal end of the delivery tube 50 thereby pushing the filter 10 until the filter 10 reaches the distal end of the delivery tube 50 to a desired location.

During deployment, the secondary struts 30 expand first to centralize or balance the filter within the vessel. When the free ends of the secondary struts emerge from the distal end of either of the delivery tubes 48 or 50, the secondary struts 30 expand to an expanded position as shown in both Figures 6a and 6b. The second arcs 42 engage the inner wall of the vessel. The second arcs 42 of the secondary struts 30 function to stabilize the attitude of filter 10 about the center of the blood vessel. When delivering through the jugular vein (Figure 6b), the filter 10 is then pushed further by the pusher wire (not shown) until it is fully deployed.

When the filter 10 is fully expanded in the vena cava, the anchoring hooks 26 of the primary struts 12 and the second arcs 42 of the secondary struts 30 are in engagement with the vessel wall. The anchoring hooks 26 of the primary struts 12 have anchored the filter 10 at the location of deployment in the vessel, preventing the filter 10 from moving with the blood flow through the vessel. As a result, the filter 10 is supported by two sets of struts that are spaced axially along the length of the filter.

Figure 7 illustrates the filter 10 fully expanded after being deployed in inferior vena cava 52. As shown, the inferior vena cava 52 has been broken away so that the filter 10 can be seen. The direction of the blood flow BF is indicated in Figure 7 by the arrow that is labeled BF. The anchoring hooks 26 at the ends of the primary struts 12 are shown as being anchored in the inner lining of the inferior vena cava 52. The anchoring hooks 26 include barbs 29 that, in one embodiment, project toward the hub 11 of the filter. The barbs 29 function to retain the filter 10 in the location of deployment.

The spring biased configuration of the primary struts 12 further causes the anchoring hooks 26 to engage the vessel wall and anchor the filter at the location of deployment. Due to the spring biased configuration of the primary struts 12, the primary struts 12 tend to dilate the vessel by abutting and expanding the vessel wall at the point or line of engagement between the strut and the vessel wall. This dilation is not shown in the Figures, but will be understood by the skilled person. After initial deployment, the pressure of the blood flow on the filter 10 contributes in maintaining the barbs 29 anchored in the inner lining of the inferior vena cava 52. As seen in Figure 7, the second arcs 42 of secondary struts 30 also have a spring biased configuration to engage with the vessel wall.

As seen in Figure 7, the hub 11 and removal hook 46 are positioned downstream from the location at which the anchoring hooks 26 are anchored in the vessel. When captured by the struts 12 and 30, thrombi remains lodged in the filter. The filter 10 along with the thrombi may then be percutaneously removed from the vena cava. When the filter 10 is to be removed, the removal hook 46 is preferably grasped by a retrieval instrument that is percutaneously introduced in the vena cava in the direction of removal hook 16 first.

Figure 8a depicts a netting configuration or pattern formed by the primary struts 12, secondary struts 30, and the hub 11 relative to radial axis R. The netting pattern shown in Figure 8a functions to catch thrombi carried in the blood stream prior to reaching the heart and lungs to prevent the possibility of a pulmonary embolism. The netting pattern is sized to catch and stop thrombi that are of a size that are undesirable to be carried in the vasculature of the patient. Due to its compacted size, the hub minimally resists blood flow.

Figure 8a depicts the netting pattern including primary struts and secondary struts at substantially equal angular space relative to each other. The netting pattern provides an even distribution between the primary and secondary struts to the blood flow, increasing the likelihood of capturing thrombi. However, as shown in Figure 8b, it is to be understood that each of the sets of primary struts 312 and secondary struts 330 may be independently spaced substantially equally at their respective portions relative to radial axis R'. For example, the secondary struts 330 may be spaced equally relative to the other secondary struts 330 and the primary struts 312 may be spaced equally relative to the other primary struts 312. As a result, the netting pattern in this embodiment shown by the cross-sectional view of the vena cava (taken along line 8-8) will have uneven or unequal spacing between the primary struts 312 and secondary struts 330.

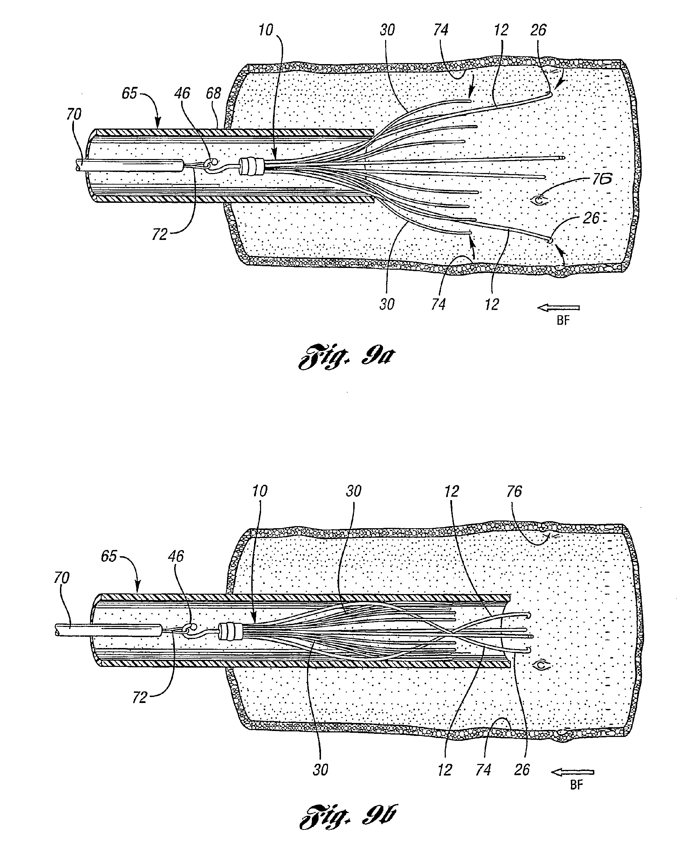

Figure 9a illustrates part of a retrieval device 65 being used in a procedure for removing the filter 10 from the inferior vena cava 52. In this example, the retrieval device 65 is percutaneously introduced into the superior vena cava via the jugular vein. In this procedure, a removal catheter or sheath 68 of the retrieval device 65 is inserted into the superior vena cava. A wire 70 having a loop snare 72 at its distal end is threaded through the removal sheath 68 and is exited through the distal end of the sheath 68. The wire 70 is then manipulated by any suitable means from the proximal end of the retrieval device such that the loop snare 72 captures the removal hook 46 of the filter 10. Using counter traction by pulling the wire 70 while pushing the sheath 68, the sheath 68 is passed over the filter 10.

As the sheath 68 passes over the filter 10, the primary struts 12 and then the secondary struts 30 engage the edge of the sheath 68 and are caus ed to pivot or undergo bend deflection at the hub 11 toward the longitudinal axis of the filter. The pivoting toward the longitudinal axis causes the ends of the struts 12 and 30 to be retracted from the vessel wall. In this way, only surface lesions 74 and small point lesions 76 on the vessel wall are created in the removal procedure. As shown, the surface lesions 74 are created by the ends of the secondary struts 30 and the small point lesions 76 are created by the anchoring hooks 26 of the primary struts 12. However, it is to be noted that any other suitable procedure may be implemented to remove the filter from the patient.

In the event that the filter 10 has stayed in the vessel for a longer period of time, the primary struts 12 may be overgrown by neovascular overgrowth of the intima layer of the vessel wall. The tendency of overgrowing of the struts is increased by the spring biased configuration of the struts 12 and the radial outward orientation of the outer end of the struts 12 in relation to the centre axis resulting in the struts dilating the vessel wall along the contact surface of the struts with the vessel wall. The intima layer overgrowing the struts 12 will increase the anchoring of the filter 10, so the struts will follow the movements of the wall, and migration of the filter is avoided. Even when the struts 12 are overgrown by intima layer, the filter 10 may be removed without any substantial damage to the vessel wall. The intima layer that has overgrown the struts will restrict the pulling forces to act parallel to the wall and thereby pulling the struts out easily, like through a canal, instead of breaking the overgrown layer. Apart from a small cut caused by the hook, there will not be any further damage, and the cut will heal easily, whereas tearing of the intima layer would be difficult to heal.

Although the embodiments of this device have been disclosed as being constructed from wire having a round cross section, it could also be cut from a tube of suitable material by laser cutting, electrical discharge machining or any other suitable process.

The primary and secondary struts can be formed from any suitable material that will result in a self-opening or self-expanding filter, such as shape memory alloys. Shape memory alloys have the desirable property of becoming rigid, that is, returning to a remembered state, when heated above a transition temperature. A shape memory alloy suitable for the present invention is Ni-Ti available under the more commonly known name Nitinol. When this material is heated above the transition temperature, the material undergoes a phase transformation from martensite to austenic, such that material returns to its remembered state. The transition temperature is dependent on the relative proportions of the alloying elements Ni and Ti and the optional inclusion of alloying additives.

In other embodiments, both the primary struts and the secondary struts are made from Nitinol with a transition temperature that is slightly below normal body temperature of humans, which is about 37°C (98.6°F). Thus, when the filter is deployed in the vena cave and exposed to normal body temperature, the alloy of the struts will transform to austenite, that is, the remembered state, which for the present invention is an expanded configuration when the filter is deployed in the blood vessel. To remove the filter, the filter is cooled to transform the material to martensite which is more ductile than austenite, making the struts more malleable. As such, the filter can be more easily collapsed and pulled into the sheath for removal.

In other embodiments, both the primary struts and the secondary struts 40 are made from Nitinol with a transition temperature that is above normal body temperature of humans, which is about 37°C (98.6° F). Thus, when the filter is deployed in the vena cave and exposed to normal body temperature, the struts are in the martensitic state so that the struts are sufficiently ductile to bend or form into a desired shape, which for the present invention is an expanded configuration. To remove the filter, the filter is heated to transform the alloy to austenite so that the filter becomes rigid and returns to a remembered state, which for the filter is a collapsed configuration.

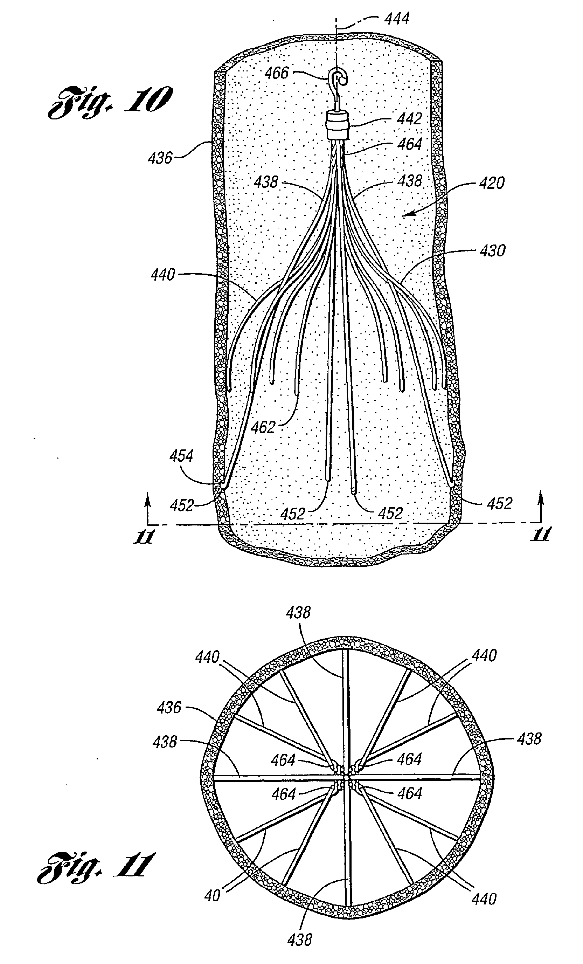

In another embodiment shown in Figures 10 and 11, a filter 420 includes four primary struts 438 and eight secondary struts 440 that extend from a hub 442. Each primary strut 438 terminates in an anchoring hook 452 with a barb 454. The primary struts 438 have sufficient spring strength such that when the filter is deployed in a vena cava 436, the anchoring hooks 452, in particular, the barbs 444, anchor into the vessel wall of the vena cava 436 to prevent the filter 420 from migrating from the delivery location. The pressure of the blood flow on the filter 420 contributes in maintaining the barbs 454 anchored in the inner lining of the vena cava 436.

A pair of secondary struts 440 are positioned between adjacent primary struts 438. Each secondary strut 440 extends from the hub 442 and terminates in a tip 462 pointing toward the central axis 444. The tips 462 are located longitudinally between the hub 442 and the anchoring hooks 454 of the primary struts 438. The connected ends of each pair of secondary struts 440 positioned between adjacent primary struts are twisted together, defining a twisted section 464.

Since the twisted sections 464 effectively stiffens each pair of secondary struts 440, thinner secondary struts may be used to provide the appropriate balancing forces to center the filter in the blood vessel. Moreover, an additional benefit of the twisted section is that they prevent the secondary struts from entangling with the primary struts. Further the size of at least some of the masks of the net or filter can be adjusted by twisting the secondary struts 440.

The secondary struts 440 can be made from the same type of material as the primary struts 438 and can be formed by the same process used to form the primary struts. However, the secondary struts may have a smaller diameter than the primary struts. To form the twisted sections 464, each pair of secondary struts 440 positioned between adjacent primary struts 438 can be twisted about each other after the struts have been attached to the hub 442. Each twisted section 464 includes one or more twists. For example, each twisted section 464 may include up to about ten twists. In certain implementations, the number of twists in each section 464 may be between about three to five twists. Increasing the number of twists increases the stiffness of the pair of secondary struts twisted about each other. The hub 442 is preferably made of the same material as the primary struts and secondary struts to minimize the possibility of galvanic corrosion.

Figure 11 illustrates a netting pattern ("net") formed by the primary struts 438, the secondary struts 440, and the hub 442. This net functions to catch thrombi carried in the blood stream to prevent the thrombi from reaching the heart and lungs, where the thrombi could cause pulmonary embolism. The net is sized to catch and stop thrombi that are of a size that are undesirable in the vasculature of the patient. As illustrated, the struts 438 have substantially equal angular spacing between the struts.

The hub 442 and a removal hook 466 attached to the hub are located downstream of the location at which the anchoring hooks 452 are anchored in the vessel 436. When captured by the struts, thrombi remain lodged in the filter 420. The filter 420 along with the thrombi may then be removed percutaneously from the vena cava. When the filter 420 is to be removed, the removal hook 466 is typically grasped by the retrieval hook that is introduced in the vena cava percutaneously.

While the present invention has been described in terms of preferred embodiments, it will be understood, of course, that the invention is not limited thereto since modifications may be made to those skilled in the art, particularly in light of the foregoing teachings and within the scope of the claims.

## Claims

1. A removable filter (10) having a collapsed state and an expanded state for capturing thrombi in a blood vessel, the filter comprising:
a plurality of primary struts (20), each primary strut in the expanded state extending from a primary strut first end to an anchoring hook (26), each primary strut having an arcuate segment extending arcuately along a longitudinal axis and linearly radially, the plurality of primary strut first ends being attached together along the longitudinal axis; and
a plurality of secondary struts (30), each secondary strut in the expanded state extending from a secondary strut first end to a free end, each secondary strut extending arcuately along the longitudinal axis, in the same longitudinal direction as the primary struts, and linearly radially, the plurality of secondary struts being attached together along the longitudinal axis, the plurality of secondary struts centralizing the filter in the expanded state in the blood vessel, **characterized in that** each arcuate segment is configured for each primary strut to cross another primary strut along the longitudinal axis in the collapsed state such that each anchoring hook faces the longitudinal axis in the collapsed state.

2. The removable filter of claim 1 further comprising:
a hub (11) configured to axially house the first ends of the plurality of primary struts; and
a retrieval hook (46) extending from the hub opposite the plurality of primary struts for removal of the filter from the blood vessel.

3. The removable filter of claim 1 wherein the arcuate segment includes a first curved portion and a second curved portion, the first curved portion extending from the first end, the second curved portion extending from the first curved portion and terminating at the anchoring hook.

4. The removable filter of claim 3 wherein the first curved portion is configured to extend radially from the longitudinal axis of the filter and the second curved portion is configured to extend radially toward the longitudinal axis of the filter.

5. Removable filter according to claim 3 or 4, wherein each primary strut, in the collapsed state, is configured to cross another primary strut along the longitudinal axis such that the arcuate segments, first curved portions or second curved portions, occupy a first diameter, whereas the anchoring hooks occupy a second, smaller diameter.

6. Removable filter according to any of the claims above, wherein the primary strut includes a distal bend formed thereon and extending outwardly radially from the longitudinal axis.

7. Removable filter according to claim 6, wherein the distal bend extends outwardly at an angle between about 0.5 degree to 2 degrees, preferably 1.0 degree.

8. Removable filter according to claim 6 or 7, wherein the distal bend is situated at a distance from the anchoring hook, which is arranged at the end of a substantially straight strut segment.

9. The removable filter of claim 3 wherein the first and second curved portions are configured to have a non-parallel relationship with the longitudinal axis of the filter.

10. Removable filter according to any of the claims above, wherein a pair of opposed primary struts are off-set by bending the strut by 1-2° at right angles to the plane of the strut.

11. The removable filter according to any of the claims above wherein each primary strut is formed of a superelastic material, stainless steel wire, Nitinol, cobalt-chromium-nickel-molybdenum-iron alloy, or cobalt-chrome alloy.

12. The removable filter according to any of the claims above wherein each secondary strut is formed of a superelastic material, stainless steel wire, Nitinol, cobalt-chromium-nickel-molybdenum-iron alloy, or cobalt-chrome alloy.

13. The removable filter according to any of the claims above wherein pairs of secondary struts are positioned between pairs of primary struts, each pair of secondary struts being twisted about each other near the connected ends of the respective secondary struts to form a twisted section.

14. The removable filter of claim 13 wherein each twisted section includes between about one and ten twists.

15. The removable filter according to any of the claims above wherein the struts are formed of shape memory alloy with a transition temperature.

16. The removable filter of claim 15 wherein the struts collapse to the collapsed state when the temperature of the struts is about equal to or greater than the transition temperature.

17. The removable filter of claim 15 wherein the struts expand to the expanded state when the temperature of the struts is about equal to or greater than the transition temperature.

## Patentansprüche

1. Entfernbarer Filter (10) mit einem kollabierten Zustand und einem expandierten Zustand zum Auffangen von Thromben in einem Blutgefäß, wobei der Filter Folgendes umfasst:
eine Vielzahl von primären Streben (20), wobei jede primäre Strebe sich im expandierten Zustand von einem ersten Ende der primären Strebe bis zu einem Verankerungshaken (26) erstreckt, wobei jede primäre Strebe ein bogenförmiges Segment aufweist, das sich bogenförmig entlang einer Längsachse und radial linear erstreckt, wobei die ersten Enden der Vielzahl von primären Streben auf der Längsachse aneinander befestigt sind; und
eine Vielzahl von sekundären Streben (30), wobei jede sekundäre Strebe sich im expandierten Zustand von einem ersten Ende der sekundären Strebe zu einem freien Ende erstreckt, wobei sich jede sekundäre Strebe bogenförmig entlang der Längsachse in derselben Längsrichtung wie die primären Streben und radial linear erstreckt, wobei die Vielzahl von sekundären Streben auf der Längsachse aneinander befestigt sind, wobei die Vielzahl von sekundären Streben den Filter im expandierten Zustand im Blutgefäß zentralisieren; **dadurch gekennzeichnet, dass** jedes bogenförmige Segment für jede primäre Strebe so konfiguriert ist, dass es eine andere primäre Strebe auf der Längsachse im kollabierten Zustand so kreuzt, dass jeder Verankerungshaken im kollabierten Zustand zur Längsachse weist.

2. Entfernbarer Filter nach Anspruch 1, ferner umfassend:
ein Ansatz (11), der so konfiguriert ist, dass er die ersten Enden der Vielzahl von primären Streben axial beherbergt; und
ein Rückholhaken (46), der sich vom Ansatz gegenüber der Vielzahl von primären Streben zum Entfernen des Filters aus dem Blutgefäß erstreckt.

3. Entfernbarer Filter nach Anspruch 1, worin das bogenförmige Segment einen ersten gekrümmten Abschnitt und einen zweiten gekrümmten Abschnitt aufweist, wobei sich der erste gekrümmte Abschnitt vom ersten Ende aus erstreckt, wobei sich der zweite gekrümmte Abschnitt vom ersten gekrümmten Abschnitt aus erstreckt und am Verankerungshaken endet.

4. Entfernbarer Filter nach Anspruch 3, worin der erste gekrümmte Abschnitt so konfiguriert ist, dass er sich radial von der Längsachse des Filters aus erstreckt und wobei der zweite gekrümmte Abschnitt so konfiguriert ist, dass er sich radial zur Längsachse des Filters erstreckt.

5. Entfernbarer Filter nach Anspruch 3 oder 4, worin jede primäre Strebe im kollabierten Zustand so konfiguriert ist, dass sie eine andere primäre Strebe auf der Längsachse so kreuzt, dass die bogenförmigen Segmente, die ersten gekrümmten Abschnitte oder die zweiten gekrümmten Abschnitte einen ersten Durchmesser besetzen, während die Verankerungshaken einen zweiten kleineren Durchmesser besetzen.

6. Entfernbarer Filter nach einem der obigen Ansprüche, worin die primäre Strebe eine darauf geformte distale Biegung aufweist, die sich von der Längsachse radial nach außen erstreckt.

7. Entfernbarer Filter nach Anspruch 6, worin sich die distale Biegung in einem Winkel zwischen ca. 0,5 Grad und 2 Grad, vorzugsweise von 1,0 Grad, nach außen erstreckt.

8. Entfernbarer Filter nach Anspruch 6 oder 7, worin sich die distale Biegung in einem Abstand vom Verankerungshaken befindet, der am Ende eines im Wesentlichen geraden Strebensegments angeordnet ist.

9. Entfernbarer Filter nach Anspruch 3, worin die ersten und zweiten gekrümmten Abschnitte so konfiguriert sind, dass sie in einer nicht-parallelen Beziehung zur Längsachse des Filters stehen.

10. Entfernbarer Filter nach einem der obigen Ansprüche, worin ein Paar gegenüberliegender primärer Streben versetzt werden, indem die Strebe rechtwinklig zur Ebene der Strebe um 1-2° gebogen wird.

11. Entfernbarer Filter nach einem der obigen Ansprüche, worin jede primäre Strebe aus einem superelastischen Material, Edelstahldraht, Nitinol, einer Kobalt-Chrom-Nickel-Molybdän-Eisen-Legierung oder einer Kobalt-Chrom-Legierung besteht.

12. Entfernbarer Filter nach einem der obigen Ansprüche, worin jede sekundäre Strebe aus einem superelastischen Material, Edelstahldraht, Nitinol, einer Kobalt-Chrom-Nickel-Molybdän-Eisen-Legierung oder einer Kobalt-Chrom-Legierung besteht.

13. Entfernbarer Filter nach einem der obigen Ansprüche, worin Paare von sekundären Streben zwischen Paaren von primären Streben positioniert sind, wobei jedes Paar von sekundären Streben in der Nähe der verbundenen Enden der jeweiligen sekundären Streben umeinander verdreht sind, um einen verdrehten Abschnitt zu formen.

14. Entfernbarer Filter nach Anspruch 13, worin jeder verdrehte Abschnitt zwischen einer und zehn Verdrehungen aufweist.

15. Entfernbarer Filter nach einem der obigen Ansprüche, worin die Streben aus einer Formgedächtnislegierung mit einer Übergangstemperatur bestehen.

16. Entfernbarer Filter nach Anspruch 15, worin die Streben in den kollabierten Zustand kollabieren, wenn die Temperatur der Streben der Übergangstemperatur etwa gleicht oder diese übersteigt.

17. Entfernbarer Filter nach Anspruch 15, worin die Streben in den expandierten Zustand expandieren, wenn die Temperatur der Streben der Übergangstemperatur etwa gleicht oder diese übersteigt.

## Revendications

1. Filtre amovible (10) qui présente un état écrasé et un état dilaté, pour capturer les caillots dans un vaisseau sanguin, le filtre comprenant :
plusieurs étrésillons primaires (20), chaque étrésillon primaire s'étendant à l'état déployé depuis une première extrémité d'étrésillon primaire jusqu'à un crochet d'ancrage (26), chaque étrésillon primaire présentant un segment cintré qui s'étend de manière cintrée le long d'un axe longitudinal et en ligne droite dans le sens radial, les premières extrémités des différents étrésillons primaires étant reliées ensemble sur leur axe longitudinal et
plusieurs étrésillons secondaires (30), chaque étrésillon secondaire s'étendant à l'état déployé depuis une première extrémité jusqu'à une extrémité libre d'étrésillon secondaire, chaque étrésillon secondaire s'étendant de manière cintrée le long de l'axe longitudinal dans la même direction longitudinale que les étrésillons primaires et en ligne droite dans le sens radial, les différents étrésillons secondaires étant reliés ensemble le long de l'axe longitudinal, les différents étrésillons secondaires centrant le filtre déployé dans le vaisseau sanguin,
**caractérisé en ce que**
chaque segment cintré de chaque étrésillon primaire est configuré de manière à croiser à l'état écrasé un autre étrésillon primaire le long de l'axe longitudinal, de telle sorte qu'à l'état écrasé, chaque crochet d'ancrage soit tourné vers l'axe longitudinal.

2. Filtre amovible selon la revendication 1, qui comprend en outre :
un moyeu (11) configuré pour loger axialement les premières extrémités des différents étrésillons primaires et
un crochet d'enlèvement (46) qui déborde du moyeu dans la direction opposée aux différents étrésillons primaires pour permettre d'enlever le filtre du vaisseau sanguin.

3. Filtre amovible selon la revendication 1, dans lequel le segment cintré comprend une première partie incurvée et une deuxième partie incurvée, la première partie incurvée débordant de la première extrémité et la deuxième partie incurvée débordant de la première partie incurvée et se terminant sur le crochet d'ancrage.

4. Filtre amovible selon la revendication 3, dans lequel la première partie incurvée est configurée de façon à déborder radialement de l'axe longitudinal du filtre, la deuxième partie incurvée étant configurée pour déborder axialement en direction de l'axe longitudinal du filtre.

5. Filtre amovible selon les revendications 3 ou 4, dans lequel chaque étrésillon primaire est configuré de manière à croiser à l'état écrasé un autre étrésillon primaire le long de l'axe longitudinal, de telle sorte que les segments cintrés, les premières parties incurvées ou les deuxièmes parties incurvées occupent un premier diamètre tandis que les crochets d'ancrage occupent un deuxième diamètre, plus petit.

6. Filtre amovible selon l'une quelconque des revendications qui précèdent, dans lequel une courbe distale qui déborde radialement vers l'extérieur de l'axe longitudinal est formée sur l'étrésillon primaire.

7. Filtre amovible selon la revendication 6, dans lequel l'extrémité distale déborde vers l'extérieur sous un angle compris entre environ 0,5 degré et 2 degrés et de préférence de 1,0 degré.

8. Filtre amovible selon les revendications 6 ou 7, dans lequel la courbe distale est située à une distance du crochet d'ancrage agencé à l'extrémité d'un segment d'étrésillon essentiellement rectiligne.

9. Filtre amovible selon la revendication 3, dans lequel la première et la deuxième partie incurvée sont configurées de manière à ne pas être parallèles à l'axe longitudinal du filtre.

10. Filtre amovible selon l'une quelconque des revendications qui précèdent, dans lequel deux étrésillons primaires opposés sont décalés par flexion de l'étrésillon de 1-2 ° à angle droit par rapport au plan de l'étrésillon.

11. Filtre amovible selon l'une quelconque des revendications qui précèdent, dans lequel chaque étrésillon primaire est constitué d'un matériau superélastique, d'un fil d'acier inoxydable, de nitinol, d'un alliage de cobalt, chrome, nickel, molybdène et fer ou d'un alliage de cobalt et chrome.

12. Filtre amovible selon l'une quelconque des revendications qui précèdent, dans lequel chaque étrésillon secondaire est formé d'un matériau superélastique, d'un fil d'acier inoxydable, de nitinol, d'un alliage de cobalt, chrome, nickel, molybdène et fer ou d'un alliage de cobalt et chrome.

13. Filtre amovible selon l'une quelconque des revendications qui précèdent, dans lequel deux étrésillons secondaires sont placés entre deux étrésillons primaires, les étrésillons secondaires de chaque paire étant torsadés l'un autour de l'autre à proximité des extrémités reliées des étrésillons secondaires correspondants, de manière à former une partie torsadée.

14. Filtre amovible selon la revendication 13, dans lequel chaque partie torsadée comprend entre environ une et dix torsions.

15. Filtre amovible selon l'une quelconque des revendications qui précèdent, dans lequel les étrésillons sont formés d'un alliage à mémoire de forme qui présente une température de transition.

16. Filtre amovible selon la revendication 15, dans lequel les étrésillons s'écrasent dans l'état écrasé lorsque la température des étrésillons est sensiblement égale ou supérieure à la température de transition.

17. Filtre amovible selon la revendication 15, dans lequel les étrésillons se déploient dans l'état déployé lorsque la température des étrésillons est sensiblement égale ou supérieure à la température de transition.
